# EUROPEAN PATENT APPLICATION

(11) **EP 0 525 958 A1**
(43) Date of publication of application: **03.02.1993**
(21) Application number: 92305249.2
(22) Date of filing: 08.06.1992
(51) Int. Cl.: A61N 1/36

(54) **Rate responsive pacemaker employing grafted sino-atrial cells**

(30) Priority: 01.08.1991 US 738887
(71) Applicant: TELECTRONICS N.V., Curaçao (AN)
(72) Inventor: Collins, Kenneth A., Neutral Bay, NSW 2080 (AU)
(74) Representative: Hackett, Sean James

(57) **Abstract**

Sino-atrial cells from a donor are implanted in a heart (103). The electrical response of the donor cells is monitored and used to adjust the pacing rate of an implanted pacemaker (101). The donor cells respond to increased cardiac stress even though the patient's natural pacing center is unable to respond to such stress.

## Description

### TECHNICAL FIELD

This invention relates generally to pacemakers and, more particularly, to an improved rate responsive pacemaker which uses grafted sino-atrial cells in order to more accurately respond to changes in cardiac stress level and to increase the pacing rate accordingly.

### DESCRIPTION OF THE PRIOR ART

Implantable pacemakers are well known in the art and have been in use for some two decades. Recently, rate responsive pacemakers have become available which can dynamically change the rate at which pacing pulses are delivered to the patient's heart when it is determined that changed stress levels are present. Thus, patients with implanted pacemakers can nonetheless climb stairs or run, and their heart rates will respond by increasing accordingly.

Prior rate responsive pacemakers use various physiological parameters in order to determine the proper increase or decrease in heart rate. For example, body motion is used in some prior pacemakers where increased body motion causes the pacemaker to increase the pacing rate, the idea being that increased body motion is a result of exercise or other movement which requires the heart to beat faster. Other parameters used by rate responsive pacemakers include the QT interval, minute volume, etc. All of these parameters, however, only indirectly reflect the required heart rate and are subject to errors. A discussion of the problems with the parameters utilized in present day rate responsive pacemakers can be found in the article, Lau P. et al., "Comparison of Exercise Performance of Six Rate Adaptive Right Ventricular Cardiac Pacemakers", Am. J. Cardiology, 1989, Vol. 63, pp. 833-838. As discussed in the Lau article, pacemakers which respond to body motion, for example, may increase the pacing rate in response to the patient riding in a car, even though the patient does not require such increased pacing rate. Many of the other parameters utilized by rate responsive pacemakers of the prior art are subject to similar errors.

A normal heart increases its pacing rate in response to catecholamines which are released, when needed, by the adrenal medulla and which circulate in the patient's blood. It would be ideal were a pacemaker able to respond to these catecholamines, However, no known technique exists for effectuating such a response.

Because the rate responsive pacemaker, when compared with the pacemaker which can only pace at a single predetermined rate, provides the advantage that the patient can lead a more normal life and can participate in a wide variety of activities, the rate responsive pacemaker is highly preferred, despite the above problems. Thus, the problem remaining in the prior art is to provide a rate responsive pacemaker which can more accurately detect the physiological parameters indicative of increased or decreased cardiac stress and can respond by increasing or decreasing the pacing rate accordingly.

### SUMMARY OF THE INVENTION

These and other problems of the prior art are overcome in accordance with the present invention which relates to a rate responsive pacemaker that utilizes a novel technique of determining when there is increased stress upon the heart, and responds thereto by increasing the pacing rate. In accordance with the invention, donor cells are implanted upon the pacemaker lead within the heart, such donor cells being selected to respond to catecholamine stimulation, such stimulation being accurately and directly proportional to increased cardiac stress. Preferably, the cells are a sino-atrial xenograft. Such cells are from the heart's natural pacing center, and therefore, exhibit electrical activity in the form of voltage pulses at an output rate which varies in response to catecholemines.

The pulse rate of such transplanted cells is monitored, and the rate of the pacemaker is adjusted according to changes thereof. The technique provides an extremely accurate measurement of changes in cardiac stress and can be used to determine when to increase or decrease the pacing rate of the pacemaker. Furthermore, the technique is subject to far fewer errors than prior techniques. Although xenograft cells are preferred, allograft cells may also be used.

### DETAILED DESCRIPTION OF THE PREFERRED EMBODIMENT

Much work has been done on the use of xenograft cells in the treatment of diabetes, for example. Xenografts are transplants of cells from one species to another. It has been shown that xenografted cells can be transplanted and, with proper drug and other treatment, may be successfully incorporated into a host without rejection by the host.

Of particular importance is the fact that fetal sino-atrial cells from different species can be transplanted and retain their activity. In this regard, see, for example, Davis M.C. and McKenzie I.F.C., "Immunological Enhancement can be Mediated by anti-la and anti-K, D Antibodies", Transplantation 1990, Vol. 29, pp. 77-79. Although the donor cells are removed from the heart of a different species, when such cells are transplanted into a human heart they still retain their electrical activity and, consequently, still respond to catecholamine stimulation. Thus, when the human heart is put under stress due to increased physical activity, catecholamines will be released into the patient's bloodstream and the donor cells will react by increasing the rate of voltage pulses. The increased voltage pulse rate may be measured by an implanted pacing lead and the pacing rate adjusted accordingly.

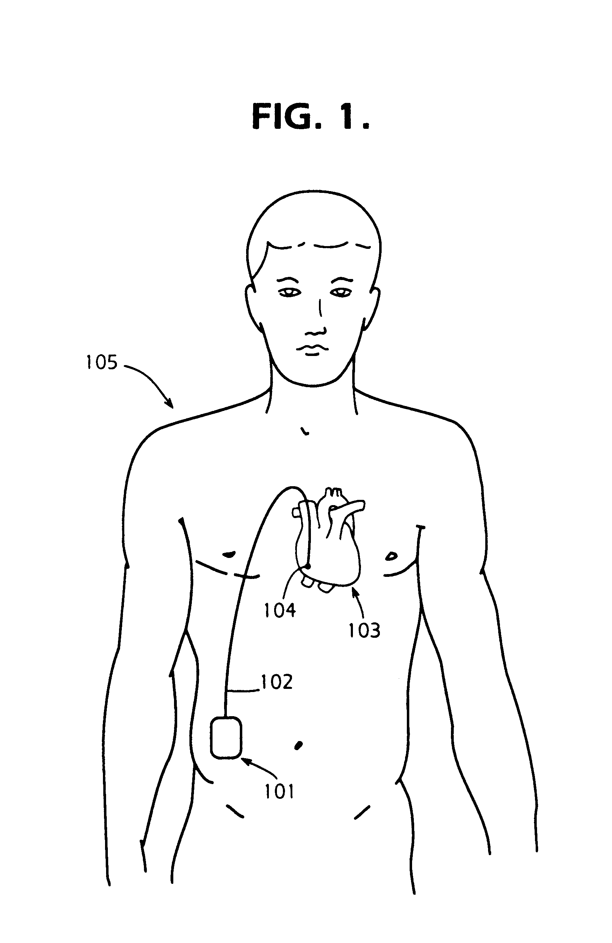

FIG. 1 shows, in conceptual form, a pulse generator (pacemaker) 101 implanted within patient 105. Electrodes 104 are positioned within the heart 103, preferably in the ventricle, and are connected by a lead 102 to the pulse generator.

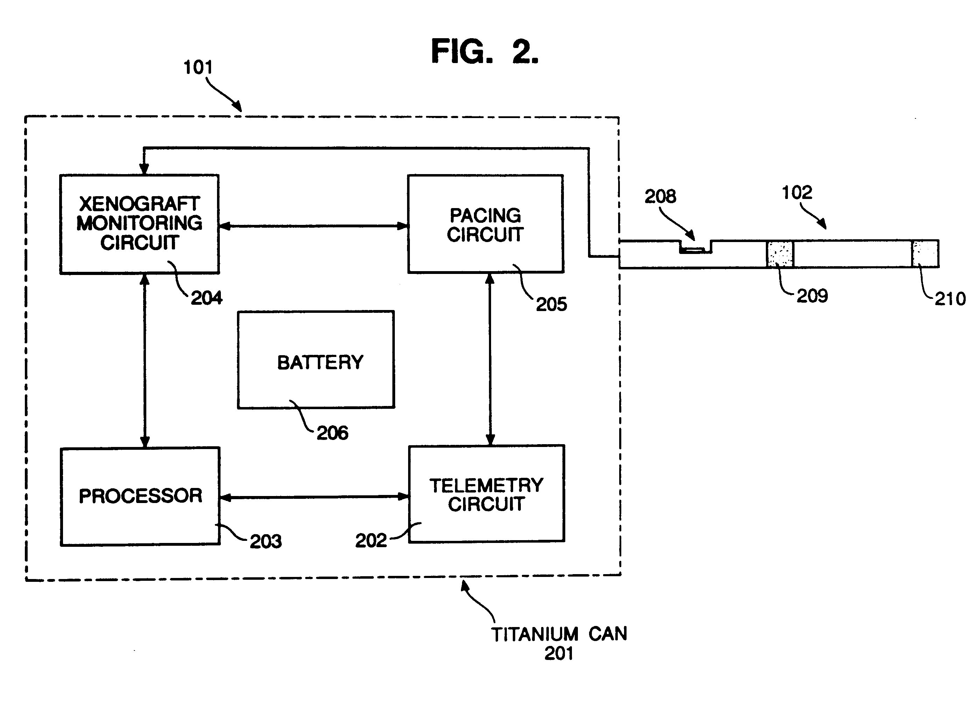

FIG. 2 shows, in conceptual block diagram form, the main components of a ventricular pacemaker 101. The arrangement of FIG. 2 includes xenograft monitoring circuit 204, pacing circuit 205, telemetry circuit 202 for allowing communication between the pacemaker and external devices, battery 206, and microprocessor 203 for control and/or management of the entire device. Tripolar lead 102 includes electrode 209 for monitoring electrocar- diac response of the heart, electrode 210 for delivering pacing pulses to the heart, and electrode 208 which is utilized for implementing the xenograft. The connections from electrodes 209 and 210 to the pacing circuit are not shown for purposes of clarity. All of these components are housed within a hermetically sealed titanium can 201 as indicated in FIG. 2 and as well known in the art.

The mass of xenograft cells implanted must be small, i.e., several fractions of a milligram, because the xenograft will rely upon passive diffusion of nutrients when it is first implanted.

Pacing circuit 205 delivers pacing pulses to the heart at a predetermined rate. The rate and timing are controlled by microprocessor 203 and the system is powered by battery 206 according to conventional techniques. Xenograft monitoring circuit 204 is connected to xenograft electrode 208 and monitors electrical activity of the xenograft. All components 202-206 are preferably linked together via a standard microprocessor bus.

When catecholamines are released into the blood, the xenograft tissue reacts by producing increased electrical activity, i.e., it increases the rate at which voltage pulses are output. Xenograft monitoring circuit 204 detects the increase of electrical activity and notifies microprocessor 203. The amount of increase in electrical activity of the xenograft is indicative of the desired amount of increase in the pacing rate of the heart. Accordingly, microprocessor 203, after determining the amount of increase in the electrical activity of the xenograft, calculates the appropriate increase in the pacing rate and notifies pacing circuit 205 accordingly. Microprocessor 203 then causes pacing circuit 205 to output pacing pulses at the appropriate increased rate.

Of course, the increased electrical activity on the xenograft surface continues to be monitored by sensor circuit 204 and the pacing rate is slowed when such electrical activity decreases. Thus, generically speaking, since the patient's heart does not respond properly to the catecholemines, a xenograft implant is utilized which does respond to the catecholamines, and the response thereof is utilized by the pacemaker to force the patient's heart to exhibit the response it should have had to the catecholamines.

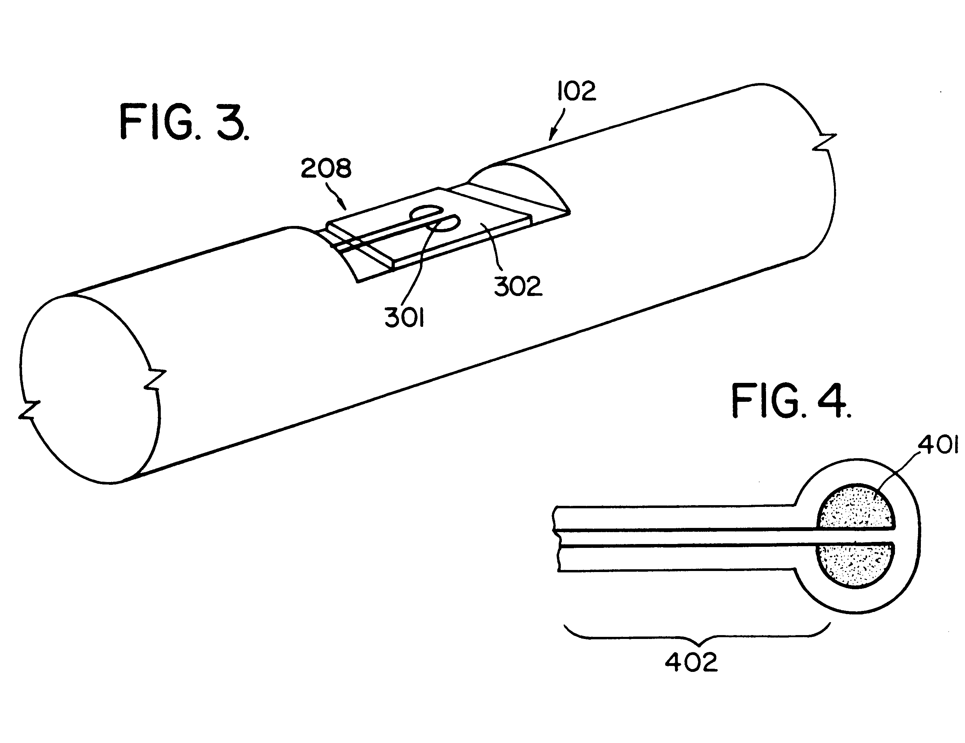

FIG. 3 shows a large view of the xenograft surface, including host plates 301. Host plates 301 are coated with cells from the xenograft, and are covered with a protective sheath of soluble biocompatible material so that the cells are protected during implant of the lead.

Host plates 301 should ideally be made from metallic glass since metallic glass has been shown to be both conductive (i.e. suitable for use in an electrode), and able to support a tissue culture. The use of metallic glasses to support tissue cultures is known in the art as demonstrated by the article of McAulan et al. "Cell Growth on Metallic Glasses: the Interaction of Amorphous Metallic Alloys with Cultured Neuronal, Osteoblast Endothelial and Fibroblast Cells", J. Biomed. Mater. Res., 1988, Vol. 22, pp. 905-917. Such a metallic glass allows xenograft cells to be cultured thereon and allows easy connection and stabilization of these cells on a cardiac electrode. Additionally, the metallic glass electrode should be mounted on a ceramic or polymeric base 302.

Immunosuppressive drugs, e.g., corticosteroids, should be used with the electrode to minimize reaction to the electrode and to further guarantee viability of the xenograft. The immunosuppressive drugs are eluted directly from ceramic base 302 in the preferred embodiment.

FIG. 4 shows another embodiment of the invention comprising lead 402 and host plates 401. The arrangement of FIG. 4 is nearly identical to that of FIG. 3 except that the xenograft is implemented on a separate lead rather than as a third electrode on the normal bipolar pacing lead. Either the arrangement of FIG. 3 or that of FIG. 4 may be used as each will monitor the electrical activity of the xenograft in response to catecholamines. It is also noted that the xenograft cells may be injected into a convenient site and the electrode implanted beside them rather than the xenograft cells being cultured directly on the electrode.

While the above describes the preferred embodiment of the invention, it is understood that various modifications may be made by those of ordinary skill in the art, such modifications being intended to be covered by the claims appended hereto.

## Claims

1. A rate responsive pacemaker for pacing a patient's heart, said rate responsive pacemaker comprising:
means implanted in the patient for delivering pacing pulses to the heart at a predetermined rate, said predetermined rate being adjustable;
a plurality of cells from a donor other than the patient, said plurality of cells being implanted in the patient and being of a type which varies its electrical activity in response to cardiac stress; and
means for monitoring the electrical activity of said plurality of cells, and for adjusting said predetermined rate in response to variations in said electrical activity of said implanted cells.

2. The pacemaker of claim 1 wherein said implanted cells are a xenograft.

3. The pacemaker of claim 1 wherein said implanted cells are an allograft.

4. The pacemaker of claim 1 wherein said plurality of cells varies its electrical activity in response to catecholamines circulating in the blood.

5. The pacemaker of claim 3 wherein said allograft is a sino-atrial allograft.

6. The pacemaker of claim 2 wherein said xenograft is a sino-atrial xenograft.

7. The pacemaker of claim 1 wherein said implanted cells are arranged on a metallic glass electrode.

8. The pacemaker of claim 7 wherein said metallic glass electrode is mounted on a ceramic or polymer base and further includes immunosuppressive agents.

9. The pacemaker of claim 7 wherein said means for delivering includes a tripolar lead.

10. In a rate responsive pacemaker, a method of pacing a patient's heart comprising the steps of:
delivering pacing pulses to the heart at a predetermined rate, said predetermined rate being adjustable;
implanting in the patient a plurality of cells from a donor other than the patient, said cells being of a type which varies its electrical activity in response to cardiac stress; and
monitoring the electrical activity of said plurality of cells, and adjusting said predetermined rate in response to variations in said electrical activity of said implanted cells.

11. The method of claim 10 wherein said implanted cells are a xenograft.

12. The pacemaker of claim 10 wherein said implanted cells are an allograft.

13. The method of claim 10 wherein said plurality of cells varies its electrical activity in response to catecholamines circulating in the blood.

14. The method of claim 12 wherein said allograft is a sino-atrial allograft.

15. The method of claim 11 wherein said xenograft is a sino-atrial xenograft.

16. The method of claim 10 wherein said implanted cells are arranged on a metallic glass electrode.

17. The method of claim 16 wherein said metallic glass electrode is mounted on a ceramic or polymer base and further includes immunosuppressive agents.

18. The method of claim 17 wherein said step of delivering includes the step of delivering pacing pulses via a tripolar lead.
